# EUROPEAN PATENT APPLICATION

(11) **EP 0 613 662 A2**
(43) Date of publication of application: **07.09.1994**
(21) Application number: 93118144.0
(22) Date of filing: 09.11.1993
(51) Int. Cl.: A61B 17/56, A61B 17/16

(54) **Spinal stabilization system and method**

(30) Priority: 09.11.1992 US 973294; 04.11.1993 US 145603
(71) Applicant: HOSPITAL FOR JOINT DISEASES ORTHOPEDIC INSTITUTE, New York New York 10003 (US)
(72) Inventor: Margulies, Joseph Y., New York, N.Y. 10003 (US)
(74) Representative: Wasmeier, Alfons, Dipl.-Ing.

(57) **Abstract**

A spinal stabilization system for fixing affected vertebrae having anterior and posterior aspects. The system comprises a plurality of rods or plates disposed over the affected vertebrae, at laest one rod or plate disposed anteriorly and at least one disposed posteriorly, and a plurality of bolts and nuts extending laterally through the affected vertebrae and attached to the rods.

A method for installing a spinal stabilization system to fix vertebrae, comprising the steps of: exposing the spine anteriorly and posteriorly to reveal a level of vertebrae, inserting a pair of beam means through the vertebrae; repeating the exposing and inserting steps for a next level of vertebrae; disposing column means over the anterior and posterior aspects of the two levels of vertebra, and connecting the ends of the beam means at each vertebrae level to the column means.

An alignment fixture for assisting in the installation of a spinal stabilization system, comprising first and second arms, each arm having first and second ends, a drill guide means and an aiming pin. The drill guide means and aiming pin are fixedly connected to the first end of the first and second arms, respectively. The second ends of the first and second arms are connected in sliding engagement with one another. The first and second arms, the drill guide means and the aiming pin are all disposed in the same plane.

## Description

### Field of the Invention

This application is a continuation-in-part of application Serial No. 07/973 294 filed November 9, 1992.

This invention relates to a system of spinal stabilization, a method for fixing vertebrae and an alignment fixture used to install said system.

### Background of the Invention

Spinal stabilization is a common method to treat pathological processes in the spine and/or sacrum. Stabilization means eliminating movement between adjacent vertebrae, and therefore restoring structural integrity to the spine. Stabilization is achieved by fusing affected vertebrae to each other. The technical objective of stabilization is to achieve a solid bony fusion. This is usually done by creating a "fracture situation" between adjacent affected vertebrae, and fixating them mechanically with metal implants. Once fixation is accomplished, bone healing occurs, creating a fusion mass, which replaces the spanned vertebrae with one solid piece of bone. The task of the implanted instrumentation is to hold fractured or injured bone surfaces rigidly together until healing and fusion occur.

Without bone fusion the stability of a mechanical construct is inadequate. The present surgical state of the art is a circumferential (anterior and posterior) approach, to rebuild the anterior and the posterior mechanical segments of the spinal column separately.

The disadvantages of the circumferential approach are several: too much metal in two different sets of devices, the construct may fail to promote stress sharing and may create stress shielding, and the construct may be too strong for osteogenic bone, and thus cut through it.

A major problem of the present day approach is what to do with poor quality bone or with spines in which adequate attachment of the construct to the bone cannot be achieved.

### Summary of the Invention

The present invention in its broadest aspect provides a single spinal stabilization system for fixing affected vertebrae having anterior and posterior aspects. The system comprises a plurality of substantially rigid, elongated column means disposed over the affected vertebrae. At least one of the column means is disposed over the anterior aspect of the affected vertebrae. At least another of the column means is disposed over the posterior aspect of the affected vertebrae. A plurality of substantially rigid, elongated beam means which extend through these affected vertebrae are attached to the column means.

Additionally, the present invention is one of its broadest aspects provides a method of installing a spinal stabilization system to fix affected vertebrae. The steps comprise exposing the spine anteriorly aned posteriorly at the level of the affected vertebra, inserting one or a pair of beam means through the affected vertebra, repeating the exposing and inserting steps for a next level of vertebra, disposing column means over the anterior and posterior aspects of the two levels of affected vertebrae, and connecting the ends of the beam means at each affected vertebra level to said column means.

Another feature of the present invention in its broadest aspect is to provide an alignment fixture which accurately place the elements of the spinal stabilization system. The alignment fixture comprises a first and secnd arm, each arm having first and second ends, a drill guide means and an aiming pin. The arm pin and drill guide means are fixedly connected to the first end of said first and second arms, respectively. The second ends of said first and second arms are connected in sliding engagement with one another. The first and second arms, the drill guide means and the aiming pin are all disposed in the same plane.

Furthermore, the present invention in still another of its broadest aspects, provides a method of installing a spinal stabilization system to fix the vertebrae of a patient using the aligning fixture which has a drill guide at one end and an aiming pin at the other end. This method comprises the steps of:
a) surgically exposing two adjacent levels of affected vertebrae anteriorly and posteriorly;
b) cleaning out the contents of the pedicle portions of the adjacent levels of affected vertebrae so as to permit sufficient movement of the drill guide within the cleaned cut pedicle portions so that the aiming pin can be positioned at the desired exit points on the anterior aspect of the adjacent levels of affected vertebrae;
c) inserting the drill guide within the cleaned out pedicle portions of the adjacent levels of affected vertebrae;
d) positioning the aiming pin at the desired exit points on the anterior aspect of the adjacent levels of affected vertebrae;
e) inserting a drilling means within the aligned drill guide disposed within the pedicle portions of the adjacent levels of affected vertebrae, and
f) drilling holes through the aligned drill guide into the affected vertebrae to the desired exit points.

The principal object of the invention is to create a rigid structure that will allow successful bony fusion by using a new concept combining anterior and posterior constructs into a single unitary structure achieved at a single surgical sitting.

Another object of the invention is to provide a more rigid construct or cage in which bone and bone graft can heal to a solid fusion.

A further object of the invention is to provide an improved solid construct, in which stress sharing is better distributed between anterior and posterior elements.

An additional object of the invention is to provide an improved method and an alignment fixture for installing such construct or cage.

### Brief Description of the Invention

Fig. 1 is a perspective view of an individual vertebra showing the threaded apertures which are formed by insertion of a pair of nut and bolt portions of the present invention;
Fig. 2 is a perspective view of two vertebrae and an intervertebral disc showing the threaded apertures which are formed by insertion of two pairs of nut and bolt portions of the present invention;
Fig. 3 is a perspective view of the implanted spinal stabilization system of the present invention;
Fig. 4A is a perspective view of a preferred embodiment of a single unassembled bolt and nut portions of the present invention;
Fig. 4B is a perspective view of a preferred embodiment of another single unassembled bolt and nut portions of the present invention for use with smaller vertebrae;
Fig. 5 is a perspective view of a single assembled bolt and nut portions attached at its ends to rods by tie wires;
Fig. 6 is a perspective view of an alignment fixture used for installing the spinal stabilization system;
Fig. 7 is a horizontal cross-sectional view of the patient and spine and a side view of the alignment fixture in its installation position adjacent the posterior and anterior aspects of the spine; and
Fig. 8 is a perspective view of the patient with a posterior incision and the alignment fixture positioned in the installation position.

### Description of the Invention

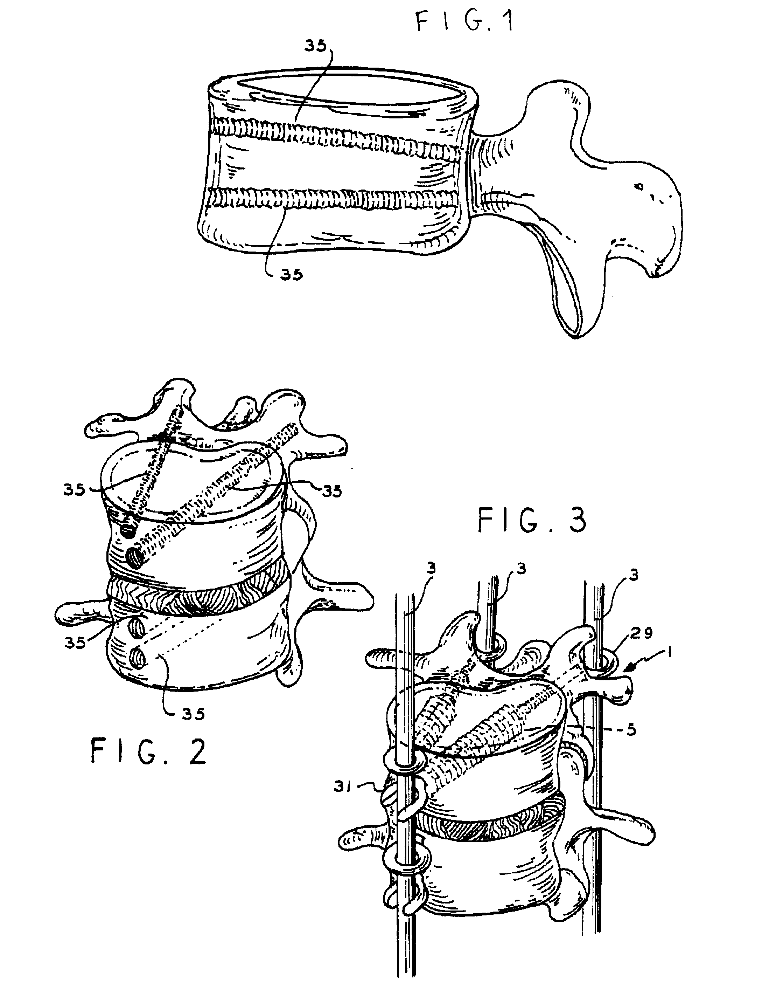

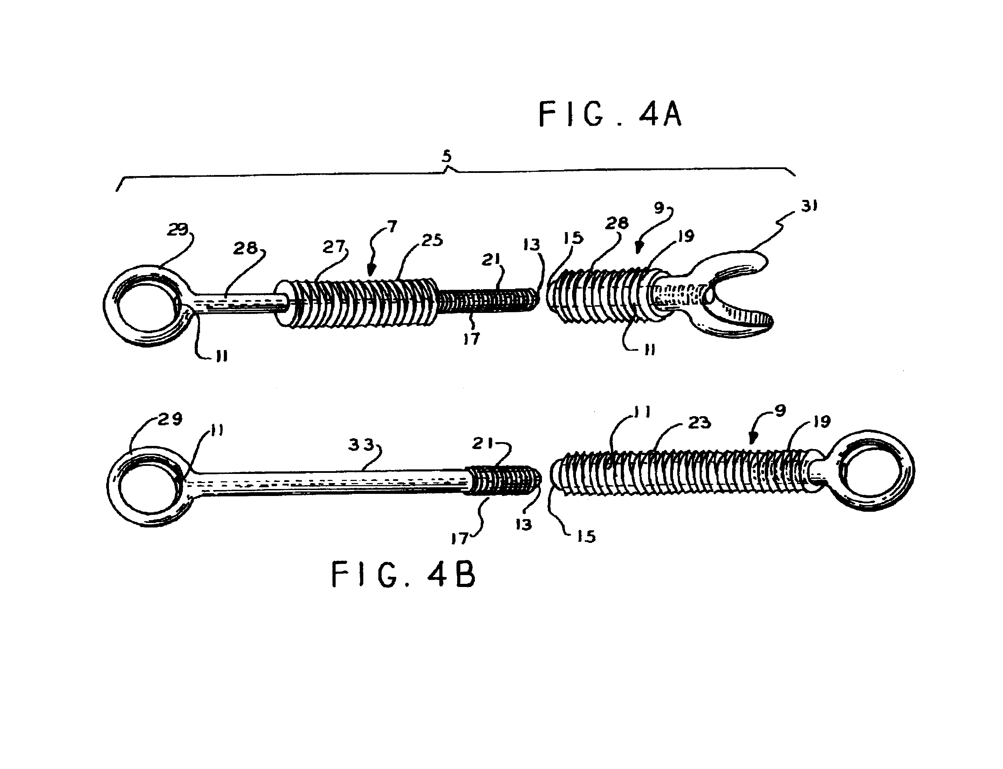

The preferred embodiment of the spinal stabilization or fixation system for treating pathological processes or fixing affected vertebrae having anterior and posterior aspects is generally designated by the reference character 1 and its complete assembly is shown in Fig. 3. An example of a pathological process in the spine having affected vertebrae is the common prolapsed invertebral disc; the vertebrae adjacent said disc are the affected vertebrae. The system comprises conventional, column means 3, three in number, each of which is a rigid elongated rod or plate, one of which is disposed over the anterior aspect of at lest two such affected vertebrae, and as shown in this figure two vertebrae, and two of which rods are disposed over the posterior aspects of the two vertebrae. These rods 3 are attached to beam means 5, four in number, each of which is a ridid elongated beam means which extends through individual vertebrae. It should be noted, however, that two or three or more rods could be used. Referring specifically to Fig. 4A, which illustrates in detail a single beam means 5 which comprises a pedicle bolt section 7 and a nut section 9, each of which sections are adapted to join each other when they are inserted through a vertebra when the system 1 is installed. Each of these bolt and nut sections 7 and 9, respectively, preferably have an aperture or cannulation 11 extending along the longitudinal axes thereof; such cannulation 11 is however optional. Each of the bolt and nut sections 7 and 9, respectively, have a cutting end portion 13 and 15, and a front portion 17 and 19, having a cylindrical periphery 21 and 23, respectively, which is externally threaded with cutting edges. The aperture 11 of the nut section 9 has a cylindrical periphery which is internally threaded for receiving and engaging the threads on the front portion 17 of the bolt section 7. The bolt section 7 also comprises a middle portion 25 having a cylindrical periphery 27 which is externally threaded with cutting edges; equal diameters are used for the middle and front portions 25 and 19, respectively. The middle portion 25 is connected to a smooth shaft portion 28. Each of the bolt and nut sections 7 and 9, respectively, have a rear portion or ends 29 and 31, respectively, for attaching the ends of said nuts and bolts 7 and 9, respectively, to said rods 3, as is best shown in Fig. 3. These ends or attachments 29 and 31 are shown as closed and open attachments, respectively, in Fig. 4A, whereas one of said beam means 5 is shown having closed attachments at both ends; after the attachments are installed a staple or a plate (not shown) can be disposed beneath the attachments.

Another preferred embodiment of the beam means 5 is shown in Fig. 4B and is utilized when the system is used for fixing smaller vertebrae. Its middle portion 33 comprises a smooth shaft portion, the diameter of which is slightly less than the diameter of the threaded portion 21 of the front portion 17. Its attachments 29 and 31 are of the closed type.

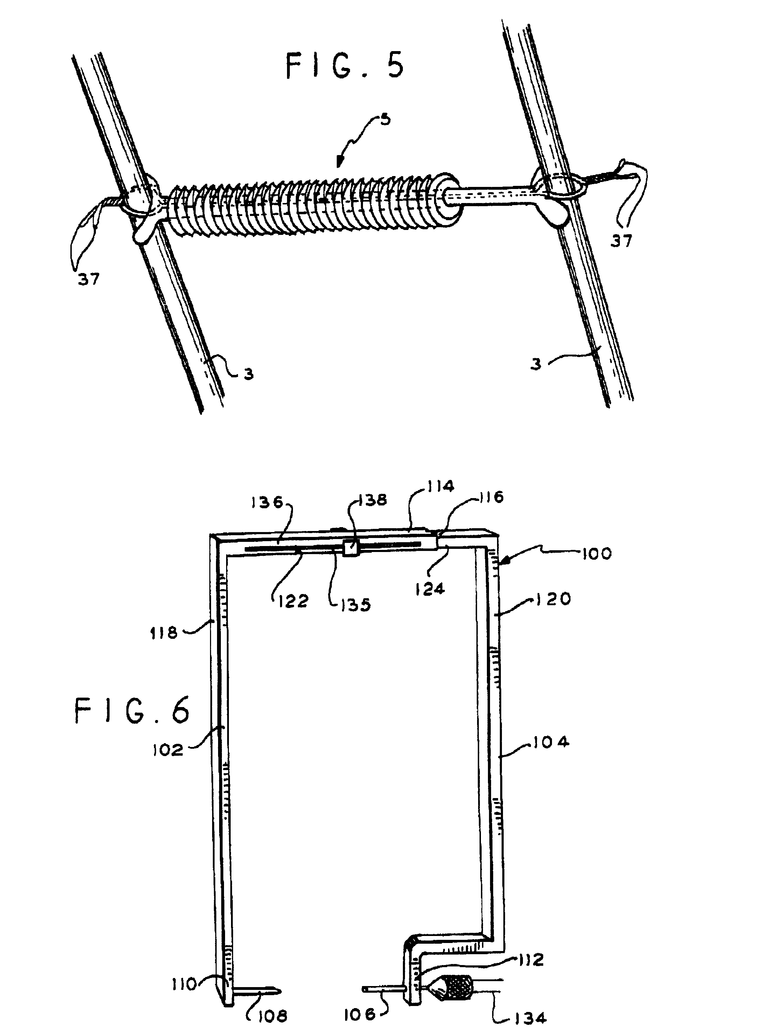

The preferred embodiment of the alignment fixture used for assisting in the installation of the previously described spinal stabilization system 1 is generally designated by the reference character 100 and its complete assembly is shown in Fig. 6. The alignment fixture 100 comprises L-shaped first and second arms 102 and 104, respectively, drill guide means 106 and an aiming pin 108, all of which are disposed in the same plane. Each of the first and second arms 102 and 104, respectively, have first and second ends 110, 112 and 114, 116, respectively. Additionally, each of the first and second arms 102 and 104, respectively, have first and second straight portions 118, 120 and 122, 124, respectively. The first portions 118 and 120 of the first and second arms 102 and 104, respectively, are disposed opposite one another as are their first ends 110 and 112. The second portion 122 is U-shaped in cross-section and has a narrow slot 135 extending across it length in side walls 136. A slide 138 extends through the slots 135 and is fixedly attached to the second end 116 of the second portion 124. Accordingly, the second portions 122 and 124 of the first and second arms 102 and 104, respectiely, are connected in sliding engagement with one another as are their second ends 114 and 116. The aiming pin 108 and the drill guide means 106 are axially aligned and fixedly disposed orthogonally on the first ends 110 and 112 of the first portions 118 and 120, respectively, of the first and second arms 102 and 104, respectively. The aiming pin 108 is generally cylindrical along its length except that its inward end is conically shaped. The drill guide means 106 comprises a hollow tube which extends inward for most of its length and extends completely through the first end 112 of the second arm 104. As discussed below, the aiming pin 108 and the drill guide means 106 on the first and second arms 102 and 104, respectively, of the alignment fixture 100, will be utilized and disposed at the anterior and posterior aspects, respectively, of the vertebrae during the initial stages of the installation of the spinal stabilization system 1. Such positioning is accomplished by moving the slide 130 appropriately thereby moving the sliding arms 102 and 104 forward or backward, until the aiming pin 108 and the drill guide 106 are properly disposed on the anterior and posterior aspects, respectively, of the vertebrae.

The surgical technique used for imnplanting the preferred system of the present invention without using the alignment fixture is generally described as follows (for fixing two levels of affected vertebrae). A portion of the spine is surgically exposed anteriorly and posteriorly simultaneously to reveal two levels of vertebrae. Then one or a pair of beam means 5 are inserted through an individual vertebra; more specifically one or two pedicle bolt sections 7 each shown as having a cannulation 11, are inserted from posterior to anterior through the vertebra so as to join its opposite bolt section 7; that is the cannulation 11 of each nut section 9 engages the threads on the front portion 17 of each bolt section 7. The route in the verterae (Fig. 1) can be prepared over a guide wire utilizing a cannula which requires usage of cannulated bolt and nut sections. The above described procedure is then repeated for the next vertibral level and then three column means or rigid elongated rods 3 are disposed over the anterior and posterior aspects of the two vertebrae and connected to these rods 3. More specifically the rear portions or ends 29 and 31, respectively, of the bolt and nut sections 7 and 9, respectively, are attached to the rods 3. Such an implanted spinal stabilization system is shown in Fig. 3. Further aspects of the present invention are as follows: the cannulated pedicle bolt 7 ac- comodates a Kirschner wire ("k-wire") which is used as a pilot locator bolt 7 and is posteriorly inserted over a properly placed k-wire (not shown) through its central aperture and through the pedicle portion of the vertebra to form a posterior portion of a threaded aperture 35; the nut 9 is similarly inserted over the k-wire anteriorly through its central aperture to form an anterior portion of the threaded aperture 35 - this aperture 35 is seen best in Fig. 2.

As seen in Fig. 2 and Fig. 3 the two beam means 5 in an individual vertebra are disposed therein in different planes and form a triangle in cross section through said beam means 5. Also as seen best in Fig. 1, the individual beam means 5 extends through the superior and inferior portions of the same vertebra. The k-wire guide inside the beam means 5 can be replaced with two tie wires 37 which are then twist locked on the rods 3, as seen in Fig. 5, after the beam sections 5 are in place in the vertebrae (not shown in said figure). These twist lock attachments serve to lock the bolt and nut sections 7 and 9, respectively, to the rods 3 to prevent back-off.

The improved surgical technique used for imnplanting the preferred system of the present invention is essentially the same as the aforedescribed original technique except that the alignment fixture 100 is used in the initial stages of the technique. This improved technique obviates the visual sighting requirement in the original technique which is used to determine the route in the vertebrae.

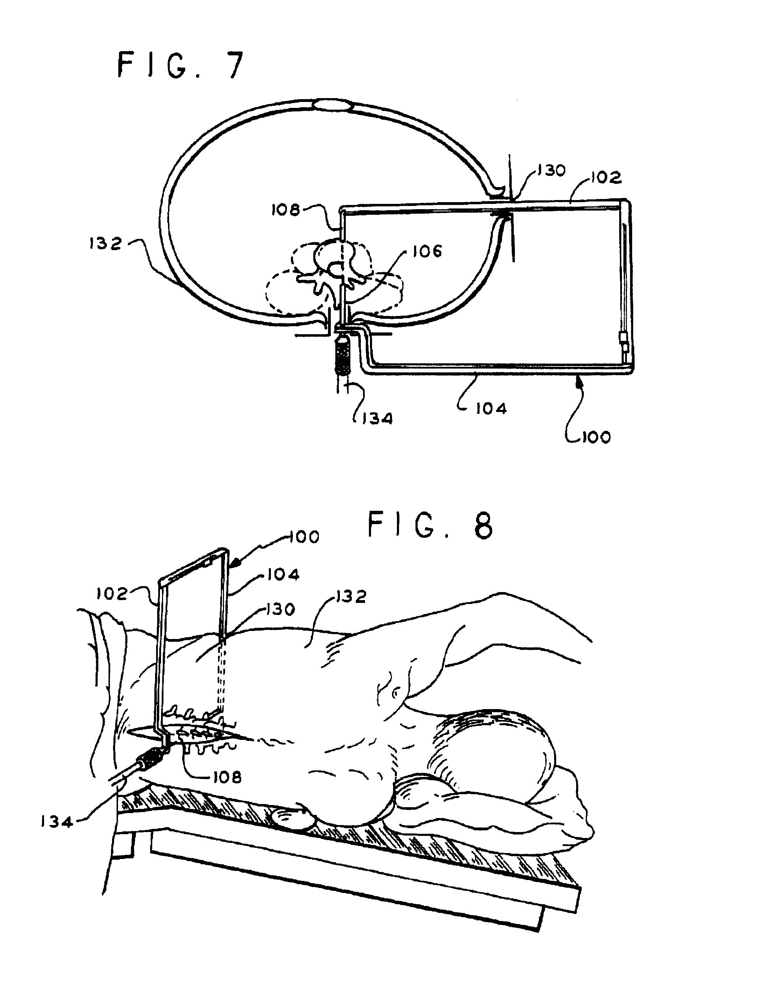

Referring now to Figs. 7 and 8, the initial stages or steps of the improved technique or process, which occur prior to the installation of the spinal stabilization system 1 are as follows:
a) surgically exposing two adjacent levels of affected vertebrea;
b) cleaning out the contents of pedical portions of the above affeced vertebrae, preferably by curetting, so as to permit sufficient movement of the drill guide means 106 (of the alignment fixture 100) within the cleaned out pedicle portions so that the aiming pin 108 (of the alignment fixture 100) can be (later) positioned at the desired exit points on the anterior aspect of the above affected vertebrae (these exit points conform to the center of the exit opening of aperture 35, as is seen best in Fig. 2);
c) making an incision 130 (Fig. 7) in the lateral aspect of a patient 132 of sufficient size to permit the aiming pin 108 and a portion of its attached arm 102 entry through the incision 130;
d) inserting the aiming pin 108 and a portion of its attached arm 102 through the incision 130 and positioning the aiming pin 108 (by moving first arm 102 inward using the slide) near the anterior aspect of one of the affected vertebrae;
e) inserting the tip of the drill guide means 106 within one of the cleaned out pedicle portions of one affected vertebra (first level);
f) positioning the aiming pin 108 at the desired exit point on the anterior aspect of said one affected vertebrae as is seen best in Fig. 7;
g) inserting the drill bit of a drill 134 (only a portion of which is shown) within the now aligned drill guide means 106 disposed within the pedicle portion of the said one affected vertebra;
h) drilling an aperture into and through the said one affected vertebra, through said drill guide means 106, which emerges at the desired exit point on the anterior aspect of said vertebra;
i) repeating steps e) through h) for the other exit point on the same affected vertebra;
j) inserting a pair of beam means 5 through the same said affected vertebra (first level);
k) repeating steps e) through j) for the second level of affected vertebra;
I) disposing three column means 3 over the anterior and posterior aspects of the two levels of affected vertebrae, and
m) connecting the ends 29 and 31 of the beam means 5 at both levels of affected vertebrae to the column means 3.

The nut section 9 has a self-tapping bone screw on its exterior aspects as does the bolt section 7. The bolt section 7 of Fig. 4A preferably has a cutting edge portion 13 around the cannulated opening 11 of 2 mm in length, an externally threaded front portion 17 of greater than between 10 mm to 30 mm in length, an externally threaded middle portion 25 of between 15 mm to 40 mm in length, and a smooth shaft portion 28 of between 20 mm to 40 mm in length. The nut section 9 of Fig. 4A preferably has an externally threaded front portion 19 of about between 10 mm to 30 mm in length, and the aperture 11 thereof has an internal machine thread to meet the machine thread in the tip 13 and front portion 17 of the bolt section 7. The pitch of the matching male and female threads are the same. The preferred materials for the bolt and nut sections 7 and 9, respectively, would be T₁-6AI-4V, or a similar material because of its mechanical strength properties and corrosion resistance; other possible materials could be a high strength biodegradable polymer such as a high molecular weight PLA. Stainless steel could even be used.

Although the present invention has been described and illustrated with respect to a preferred embodiment; it is not to be so limited since modifications and changes can be made therein which are within the full intended scope of the invention.

## Claims

1. A spinal stabilization system for fixing affected vertebrae having anterior and posterior aspects, said system comprising:
a plurality of substantially rigid, elongated column means for being disposed over the affected vertebrae, at least one of said column means for being disposed over the anterior aspect of said affected vertebrae and at least another of said column means for being disposed over the posterior aspect of said affected vertebrae, and
a plurality of substantially rigid, elongated beam means having a longitudinal axis for extending through said affected vertebrae and for attaching said column means to said beam means.

2. The system as recited in claim 1, wherein each of said beam means has an aperture extending along its longitudinal axis.

3. The system as recited in claim 1, wherein said beams extending through the same individual said affected vertebrae are disposed therein in different planes.

4. The system as recited in claim 1, wherein said beam means extend from the posterior aspect to the anterior aspect of said affected vertebrae.

5. The system as recited in claim 1, wherein column means comprise rods or plates and wherein said plurality of column means comprise at least two column means.

6. The system as recited in claim 1, wherein said column means has a cross section and wherein the cross section of said column means through said beam means form a triangular configuration.

7. The system as recited in claim 1, wherein said vertebrae have superior and inferior rim portions and wherein individual beam means extend through said superior and inferior rim portions of a common said affected vertebrae.

8. The system as recited in claim 1, wherein each said beam means comprises separate bolt and nut sections having longitudinal axes which sections join each other when said sections are inserted into and through an individual said affected vertebra.

9. The system as recited in claim 8, wherein each of said bolt and nut sections has an aperture extending along the longitudinal axes thereof.

10. The system as recited in claim 8, wherein each of said bolt and nut sections comprises a cutting end portion and a front portion having an externally threaded cylindrical periphery with cutting edges, said aperture of said nut section having an internally threaded cylindrical periphery for engaging said cutting edges of said externally threaded cylindrical periphery of said front portion of said bolt section.

11. The system as recited in claim 10, wherein said bolt sections comprise a middle portion having an externally threaded periphery with cutting edges.

12. The system as recited in claim 10, wherein each of said bolt and nut sections comprise a rear portion for attaching said rear portions of said bolt and nut sections to said column means.

13. The system as recited in claim 10, further comprising wire means extending through said aperture of said beam means for attaching said column means to said beam means.

14. The system as recited in claim 11, wherein the diameter of the cylindrical peripheries of the middle portion of said bolt section and said front portion of said nut section are substantially equal.

15. The system as recited in claim 8, wherein said vertebrae have pedicle portions and said bolt sections extend through the pedicle portions of said affected vertebrae.

16. The system as recited in claim 8, wherein said bolt sections and nut sections extend from the posterior and anterior aspects, respectively, of said affected vertebrae, and towards each other.

17. A spinal stabilization system for fixing affected vertebrae having anterior and posterior aspects, said system comprising:
at least two rods or plates being disposed over the affected vertebrae, at least one of said rods or plates being disposed over the anterior aspect of said affected vertebrae and at least one said rod or plate being disposed over the posterior aspect of said affected vertebrae,
a plurality of elongated beam means having a longitudinally axis extending through said affected vertebrae and attached to said rods or plates, at least two of said beams extending through different planes of the same individual said affected vertebra, and
each of said beam means comprising separate bolt and nut sections, said nut sections having longitudinal axes and an aperture, said aperture extending along its longitudinal axes, each said sections comprising a cutting end portion and a front portion having a cylindrical periphery externally threaded with cutting edges, the aperture of said nut section having a cylindrical periphery internally threaded for engaging said externally threaded cutting edges of said first portion of said bolt section.

18. A method of installing a spinal stabilization system to fix affected vertebrae comprising the steps of:
exposing the spine anteriorly and posteriorly to reveal a level of affected vertebra,
inserting a pair of beam means through said affected vertebra,
repeating the exposing and inserting steps for a next level of affected vertebra,
disposing column means over the anterior and posterior aspects of the two levels of affected vertebrae, and connecting the ends of the beam means of each vertebra level to said column means.

19. A method as recited in claim 18, wherein the step of exposing the spine reveals a level of vertebra including its pedicle portion.

20. A method as recited in claim 19, wherein the step of inserting comprises inserting a pair of beams posteriorly through the pedicle portions of said vertebra and anteriorly through said vertebra.

21. A method as recited in claim 20, wherein the beam means comprises pedicle bolt sections and nut sections.

22. A method as recited in claim 21, wherein the beam means have cannulation.

23. A method as recited in claim 22, wherein the column means comprise rigid elongated rod-(s).

24. A method of installing a spinal stabilization system to fix affected vertebrae comprising the steps of:
exposing the spine anteriorly and posteriorly to reveal a level of affected vertebra including its pedicle portions,
inserting a pair of pedicle bolt sections posteriorly through the pedicle portion of said affected vertebra and a pair of nut sections having cannulations anteriorly through said sections,
repeating the exposing and inserting steps for a next level of affected vertebra,
disposing rigid elongated rods over the anterior and posterior aspects of the two levels of affected vertebrae, and
connecting the ends of the bolt and nut sections at each affected vertebra to said rigid elongated rods.

25. A method of installing a spinal stabilization system to fix affected vertebrae of a patient using an aligning fixture having a drill guide at one end and an aiming pin at the other end, comprising the steps of:
a) surgically exposing at least one level of affected vertebra anteriorly and posteriorly,
b) cleaning out the contents of the pedicle portions of the level of affected vertebra so as to permit sufficient movement of the drill guide within the cleaned out pedicle portions so that said aiming pin can be positioned at the desired exit points on the anterior aspect of said level of affected vertebra,
c) inserting said drill guide within the cleaned out pedicle portions of the level of affected vertebra,
d) positioning said aiming pin at the desired exit points on the anterior aspect of said level of affected vertebra,
e) inserting a drilling means within the aligned drill guide disposed within said pedicle portions of the level of affected vertebra, and
f) drilling holes through said aligned drill guide into said affected vertebra to said desired exit points.

26. The method as recited in claim 25, further comprising the steps of:
g) inserting a pair of beam means through said level of affected vertebra,
h) repeating steps a) through g) for the other level of affected vertebra,
i) disposing column means over the anterior and posterior aspect of the two levels of affected vertebrae, and
j) connecting the ends of the beam means of each vertebra level to the column means.

27. A method of installing a spinal stabilzation system to fix affected vertebrae of a patient using an aligning fixture having a drill guide at one end and an aiming pin at the other end, comprising the steps of:
a) surgically exposing two adjacent level of affected vertebrae anteriorly and posteriorly,
b) cleaning out the contents of the pedicle portions of the adjacent levels of affected vertebrae so as to permit sufficient movement of the drill guide within the cleaned out pedicle portions so that said aiming pin can be positioned at the desired exit points on the anterior aspect of said adjacent levels of affected vertebrae,
c) inserting said drill guide within the cleaned out pedical portions of the adjacent levels of affected vertebrae,
d) positioning said aiming pin at the desired exit points on the anterior aspect of said adjacent levels of affected vertebrae,
e) inserting a drilling means within the aligned drill guide disposed within said pedicle portions of the adjacent levels of affected vertebrae, and
f) drilling holes through said aligned drill guide into said affected vertebrae to said desired exit points.

28. A method as recited in claim 27, further comprising after step c):
1) making an incision in the lateral aspect of a patient of sufficient size to permit the aiming pin and a portion of its attached arm entry therethrough, and
2) inserting said aiming pin and a portion of its attached arm through said incision and disposing the aiming pin adjacent said anterior aspect of said adjacent levels of affected vertebrae.

29. A method as recited in claim 27, wherein said cleaning is performed by curetting the pedicle portions of the adjacent levels of affected vertebrae.

30. A method of installing a spinal stabilization system to fix affected vertebrae of a patient using an aligning fixture having a drill guide at one end and an aiming pin at the other end, comprising the steps of:
a) surgically exposing two adjacent levels of affected vertebrae anteriorly and posteriorly,
b) cleaning out the contents of the pedicle portions of the adjacent levels of affected vertebrae so as to permit sufficient movement of the drill guide within the cleaned out pedicle portions so that said aiming pin can be positioned at the desired exit points on the anterior aspect of said adjacent levels of affected vertebrae,
c) inserting said drill guide within the cleaned out pedicle portions of the adjacent levels of affected vertebrae,
d) positioning said aiming pin at the desired exit points on the anterior aspect of said adjacent levels of affected vertebrae,
e) inserting a drilling means within the aligned drill guide disposed within said pedicle portions of the adjacent levels of affected vertebrae,
f) drilling holes through said aligned drill guide into said affected vertebrae to said desired exit points,
g) inserting a pair of beam means through each of said levels of affected vertebrae,
h) disposing column means over the anterior and posterior aspects of the two levels of affected vertebrae, and
i) connecting the ends of the beam means at each affected vertebra level to said column means.

31. An alignment fixture for assisting in the installation of a spinal stabilization system, comprising
a first arm,
a second arm,
each arm having first and second ends, drill guide means, and an aiming pin,
said aiming pin and said drill guide means fixedly connected to the first end of said first and second arms, respectively,
said second ends of said first and second arms being connected in sliding engagement with one another, and
said first and second arms, said drill guide means and said aiming pin being disposed in the same plane.

32. An alignment fixture as recited in claim 31, wherein said drill guide means and aiming pin being disposed orthogonally on said first and second arm, respectively.

33. An alignment fixture as recited in claim 32, wherein said drill guide means and aiming pin being axially aligned.

34. An alignment fixture as recited in clam 33, wherein each of said first and second arms having first and second portions, said first portions being disposed opposite one another, and said second portions being connected together in sliding engagement.

35. An alignment fixture as recited in claim 31, wherein said first and second arms being disposed at the anterior and posterior aspects, respectively, of said vertebrae during the initial stage of the installation of said system.
